# EUROPEAN PATENT SPECIFICATION

(11) **EP 3 919 097 B1**
(45) Date of publication and mention of the grant of the patent: **16.10.2024**
(21) Application number: 19913134.3
(22) Date of filing: 30.08.2019
(51) Int. Cl.: A61M 1/34, B01D 15/10, B01D 15/22

(54) **ENCAPSULATION STRUCTURE OF BLOOD PLASMA ADSORBER AND ADSORBENT THEREOF**
EINKAPSELUNGSSTRUKTUR EINES BLUTPLASMA-ADSORBERS UND ADSORPTIONSMITTEL DAFÜR
STRUCTURE D'ENCAPSULATION D'UN ADSORBEUR DE PLASMA SANGUIN ET DE SON ADSORBANT

(30) Priority: 29.01.2019 CN 201910083595
(43) Date of publication of application: 08.12.2021
(73) Proprietor: Guangzhou Koncen Bioscience Co., Ltd., Guangzhou, Guangdong 510660 (CN)
(72) Inventor: YANG, Zhenggen, Guangzhou, Guangdong 510660 (CN); NIU, Yuewei, Guangzhou, Guangdong 510660 (CN); ZHANG, Haizhen, Guangzhou, Guangdong 510660 (CN); CHEN, Xiaoyuan, Guangzhou, Guangdong 510660 (CN)
(74) Representative: Scholl, Matthias
(86) International application number: PCT/CN2019/103513
(87) International publication number: WO 2020/155618

(56) References cited:
- CN-A- 105 107 044
- CN-A- 105 251 074
- CN-A- 109 092 263
- CN-A- 109 663 162
- CN-U- 203 647 773
- CN-U- 203 647 773
- CN-U- 204 699 119
- CN-U- 206 526 327
- JP-A- 2011 056 011
- US-A1- 2009 112 146

## Description

The disclosure relates to the technical field of medical apparatus and instruments, and more particularly to a plasma adsorber and a packaging structure of an adsorbent of the plasma adsorber.

A plasma adsorption process refers to separating visible components (blood cells and blood platelets) of blood from plasma as the blood is extracted to a plasma separator, conveying the visible components back into the body of a patient, feeding the plasma into a plasma adsorber to adsorb and eliminate some special substances in the plasma by using an adsorbent, and finally conveying the adsorbed plasma back again to the body of the patient. As far as a conventional plasma adsorber is concerned, the liquid flow such as plasma or a buffer solution passing through the adsorbent medium will generate a pressure difference thereby forming a column pressure, such that the filter part is subjected to a traction force along the direction of the liquid flow and tends to break of fall off. As a result, the adsorbent enters into the body of the patient along with the liquid flow and leads to a severe medical accident. JP2011056011A discloses an adsorbent packed column. The column includes: a casing for filling the adsorbent, the casing having a cylindrical shape, an open end, and a stepped portion; a cap fixed at an end of the casing; a filter positioned in contact with the stepped portion to prevent leakage of the adsorbent; and an O-ring disposed between the cap and the filter. CN109092263A recites a low-density lipoprotein adsorption column. The adsorption column includes a container, and an adsorbent filled in the container. The container includes a blood inlet, a blood outlet, and a filter net that prevents the leakage of the adsorbent.

The disclosure provides a plasma adsorber and a packaging structure of an adsorbent of the plasma adsorber. The packaging structure of the adsorbent can prevent the filter part from being broken or falling off and prevent the adsorbent from entering into the body of the patient, such that potential safety hazards are eliminated. Thus, the plasma adsorber adopting the packaging structure of the adsorbent can complete the plasma adsorption process safely and reliably.

The following technical solution are adopted.

On the one hand, the disclosure provides a packaging structure of the adsorbent, comprising an adsorption body, a filter part and a first cover body; the adsorption body comprises an adsorption cavity which is used for accommodating the adsorbent and comprises an opening; the first cover body comprises an abutting surface disposed toward the filter part and is hermetically matchable with the opening to abut the abutting surface with the filter part; or the filter part is disposed on the abutting surface, and the filter part is attached to the abutting surface.

When the packaging structure of the adsorbent is used, the adsorption cavity is filled with the adsorbent which is packaged in the adsorption cavity by using the filter part, and the filter part and the adsorption body are kept relatively fixed by hermetic fit of the first cover body and the opening of the adsorption body. When the liquid flow flows through, the filter part can be kept stably, such that the adsorbent does not flow out along with flowing of the liquid flow reliably. Meanwhile, when the first cover body is hermetically matched with the opening of the adsorption body, the abutting surface of the first cover body is abutted against and attached to the filter part or the filter part is disposed on the abutting surface, such that the filter part is disposed by being attached to the abutting surface, and then the first cover body is hermetically matched with the opening of the adsorption body. Therefore, even though the filter part is subjected to the traction force along the direction of the liquid flow, the abutting surface further can support the filter part, such that the traction force to the filter part can be counteracted or alleviated, the filter part can be prevented from being broken or falling off and the adsorbent is prevented from entering into the body of the patient, such that potential safety hazards are eliminated.

The technical schemes are further described as follows.

In one of embodiments, the first cover body is further provided with a blood nozzle communicating with the adsorption cavity, and the abutting surface is provided with a diversion channel communicating with the blood nozzle. Thus, liquid flows in the parts on the abutting surface are guided to the blood nozzle via the diversion channel, such that the liquid flows can flow in or out smoothly via the blood nozzle.

In one of embodiments, the abutting surface is provided with at least two oppositely disposed diversion parts at intervals; adjacent two diversion parts are matched to form a runner, the runners communicate with each other to form the diversion channel, and the diversion part abuts against the filter part when the first cover body is hermetically matched with the adsorption body. Thus, the liquid flows are drained to the blood nozzle via the diversion channel formed by the runners communicating with each other.

In one of embodiments, the diversion part comprises a diversion seat disposed on the abutting surface and a diversion section disposed on the diversion seat. Thus, adjacent diversion seats are matched with corresponding adjacent diversion sections to form the runners.

In one of embodiments, the diversion part is used for abutting against the filter part and the diversion part is in linear contact with the filter part. Thus, the contact area between the diversion part and the filter part can be reduced and the risk that the liquid flows are left in a dead angle of a gap is reduced.

In one of embodiments, the packaging structure of the adsorbent further comprises a second cover body which is hermetically matched with the first cover body and seals the blood nozzle. Thus, the blood nozzle is sealed by using the second cover body and is prevented from being in contact with external air or foreign matters in a non-used state, thereby preventing the adsorbent from being polluted.

In one of embodiments, the first cover body comprises a first mounting cavity, a bottom wall of the first mounting cavity is provided with the blood nozzle and connectors oppositely disposed on the outer wall of the blood nozzle at intervals, and the connectors are matched with the outer wall of the blood nozzle to form a second mounting cavity where the second cover body is mounted. Thus, the second cover body and the first cover body can be matched to seal the blood nozzle.

In one of embodiments, the packaging structure of the adsorbent further comprises a sealing part, the sealing part is disposed in the adsorption cavity, and the sealing part, the first cover body and the filter part are matchable with each other to form a sealing structure for sealing the opening. Thus, the sealing performance of the packaging structure is further improved by using the sealing structure, so that the adsorbent is prevented from being polluted.

In one of embodiments, an outer wall of the first cover body is provided with a first locking portion, and an inner wall of the adsorption cavity is provided with a second locking portion in locking fit with the first locking portion. Thus, the first cover body can be prevented from rotating or axially playing relative to the adsorption body during use.

On the other hand, the disclosure discloses a plasma adsorber, comprising the packaging structure of the adsorbent.

When the plasma adsorber is used, the adsorption cavity is filled with the adsorbent which is packaged in the adsorption cavity by using the filter part, and the filter part and the adsorption body are kept relatively fixed by hermetic fit of the first cover body and the opening of the adsorption body. When the liquid flow flows through, the filter part can be kept stably, such that the adsorbent does not flow out along with flowing of the liquid flow reliably. Meanwhile, when the first cover body is hermetically matched with the opening of the adsorption body, the abutting surface of the first cover body is abutted against and attached to the filter part or the filter part is disposed on the abutting surface, such that the filter part is disposed by being attached to the abutting surface, and then the first cover body is hermetically matched with the opening of the adsorption body. Therefore, even though the filter part is subjected to the traction force along the direction of the liquid flow, the abutting surface further can support the filter part, such that the traction force to the filter part can be counteracted or alleviated, the filter part can be prevented from being broken or falling off and the adsorbent is prevented from entering into the body of the patient, such that potential safety hazards are eliminated. Therefore, the plasma adsorber can complete the plasma adsorption process safely and reliably.
FIG. 1 is a structural schematic diagram of the plasma adsorber of one embodiment;
FIG. 2 is a structural schematic diagram of the the first cover body of the plasma adsorber of FIG. 1;
FIG. 3 is a partial enlarged drawing of A of the plasma adsorber in FIG. 2;
FIG. 4 is a structural schematic diagram of the second cover body of the plasma adsorber of FIG. 1;
FIG. 5 is a structural schematic diagram of the the adsorption body of the plasma adsorber of FIG. 1 .

In the drawings, the following reference numbers are used:

100. Adsorption body; 110. Adsorption cavity; 111. Opening; 120. Fill opening; 130. First step groove; 140. Second step groove; 200. Filter part; 210. Net rack; 300. First cover body; 310. Abutting surface; 320. Blood nozzle; 330. Diversion channel; 340. Diversion part; 341. Diversion seat; 342. Diversion section; 343. Notch; 350. First mounting cavity; 360. Connector; 370. Second mounting cavity; 380. Reinforcing rib; 390. Sealing groove; 400. Second cover body; 410. First sealing body; 420. First sealing cavity; 430. Second sealing body; 440. Second sealing cavity; 500. Sealing part.

To further illustrate, embodiments detailing a plasma adsorber and a packaging structure of an adsorbent of the plasma adsorber are described below. It should be noted that the following embodiments are intended to describe and not to limit the disclosure. The scope of the invention is delimited by the appended set of claims.

It should be noted that when a component is "disposed on" or "fixed on" another component, it can be directly disposed or fixed on another component or there can be an intermediate component therebetween. When a component is "fixed" to another component or "fixedly connected" to another component, it can be fixed in a detachable or non detachable way. When a component is "connected" or "rotationally connected" to another component, it can be directly connected to another component or there may be an intermediate component therebetween. The terms "vertical", "horizontal", "left", "right", "up", and "down" used in this disclosure are only for the purpose of illustration, and do not mean that they are the only mode of implementation.

Unless otherwise defined, all technical and scientific terms used herein have the same meanings as those commonly understood by those skilled in the technical field of the disclosure. The terms used in the specification of the disclosure are only for the purpose of describing the specific implementation mode, not for the purpose of restricting the disclosure. The term "and / or" as used herein includes any and all combinations of one or more related items.

The "first", "second", and "third" in the disclosure do not represent the specific quantity and order, but are only used to distinguish names.

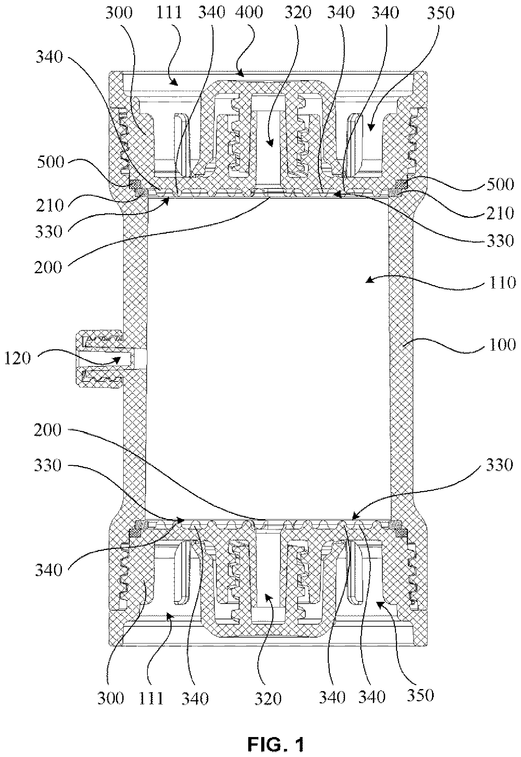

As shown in FIG. 1, in an embodiment, the disclosure provides a packaging structure of an adsorbent, comprising an adsorption body 100, a filter part 200 and a first cover body 300; the adsorption body 100 comprises an adsorption cavity 110 which is used for accommodating the adsorbent and comprises an opening 111; the first cover body 300 comprises an abutting surface 310 disposed toward the filter part 200 and is hermetically matchable with the opening 111 to abut the abutting surface 310 with the filter part 200; or the filter part 200 is disposed on the abutting surface 310, and the filter part 200 is attached to the abutting surface 310.

When the packaging structure of the adsorbent of the embodiment is used, the adsorption cavity 110 is filled with the adsorbent which is packaged in the adsorption cavity 110 by using the filter part 200, and the filter part 200 and the adsorption body 100 are kept relatively fixed by hermetic fit of the first cover body 300 and the opening 111 of the adsorption body 100. When the liquid flow flows through, the filter part 200 can be kept stably, such that the adsorbent does not flow out along with flowing of the liquid flow reliably. Meanwhile, when the first cover body 300 is hermetically matched with the opening 111 of the adsorption body 100, the abutting surface 310 of the first cover body 300 is abutted against and attached to the filter part 200 or the filter part 200 is disposed on the abutting surface 310, such that the filter part 200 is disposed by being attached to the abutting surface 310, and then the first cover body 300 is hermetically matched with the opening 111 of the adsorption body 100. Therefore, even though the filter part 200 is subjected to the traction force along the direction of the liquid flow, the abutting surface 310 further can support the filter part 200, such that the traction force to the filter part 200 can be counteracted or alleviated, the filter part 200 can be prevented from being broken or falling off and the adsorbent is prevented from entering into the body of the patient, such that potential safety hazards are eliminated.

It should be noted that the filter part 200 of the embodiment can be a filter screen, a filter membrane or other elements capable of filtering the adsorbent, so that the adsorbent does not flow out of the adsorption cavity 110 along with the liquid flows. Meanwhile, a bore diameter of a filter hole of the filter part 200 is smaller than a grain size of adsorbent filler. In order to fill the adsorption cavity 110 with the adsorbent, a filler hole 120 communicating with the adsorption cavity 110 can be further formed in a side wall of the adsorption body 100, and the filler hole 120 is sealed by using a sealing part such as a screw cap after filling. After the filter part 200 is disposed in the adsorption cavity 110 first, the first cover body 300 is hermetically matched with the opening 111, such that the abutting surface 310 abuts against the filter part 200 to support the filter part 200; the filter part 200 can be also directly disposed on the abutting surface 310, for example, the filter part 200 is attached to the abutting surface 310 in a bonding or clamping mode, such that the filter part 200 can be supported by the abutting surface 310, and then the first cover body 300, together with the filter part 200, is assembled with the adsorption body 100 and assembly and installation are achieved by hermetically matching the first cover body 300 with the opening 111.

As shown in FIG. 1, the filter part 200 is disposed in the adsorption cavity 110, and the filter part 200 can be directly connected with the inner wall of the adsorption cavity 110; and the filter part 200 can be also disposed in the adsorption cavity 110 after being disposed in a frame. In an embodiment, the filter part 200 is disposed as the filter screen, the filter screen is welded in the net rack 210 by way of rubber coating injection molding, ultrasonic or hot melting, and the inner wall of the adsorption cavity 110 is provided with the groove or the step groove where the net rack 210 is mounted. Thus, the filter part 200 can be simply and conveniently disposed in the adsorption cavity 110.

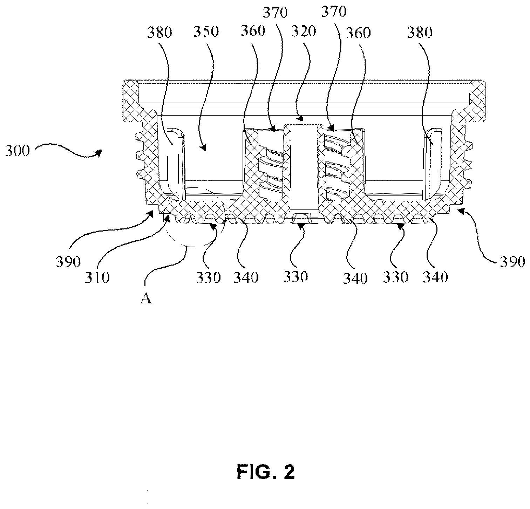

As shown in FIG. 1 and FIG. 2, the first cover body 300 is hermetically matched with the adsorption body 100, which can be achieved by either a clamping fit manner or a threaded fit manner. In an embodiment, the outer wall of the first cover body 300 is provided with an external thread, an internal thread is correspondingly disposed on the inner wall of the adsorption cavity 110, and the first cover body 300 is screwed into the adsorption cavity 110 by threaded fit of the external thread and the internal thread to achieve hermetic fit of the first cover body 300 and the adsorption body 100, such that the abutting surface 310 of the first cover body 300 can be attached to the filter part 200 to provide a supporting force to the filter part 200, thereby avoiding the problem that the filter part 200 is broken or falls off.

In an embodiment, the outer wall of the first cover body 300 is provided with the first locking portion (not shown), and the inner wall of the adsorption cavity 110 is provided with the second locking portion (not shown) in locking fit with the first locking portion. Thus, the first cover body 300 and the adsorption body 100 can be kept relatively fixed stably by means of locking fit between the first locking portion and the second locking portion to prevent the first cover body 300 from rotating or axially playing relative to the adsorption body 100 in a using process, such that the filter screen is prevented from moving, a gap between the filter screen and the adsorption cavity 110 is prevented, and the adsorbent is prevented from flowing out from the adsorption cavity 110. Locking fit between the first locking portion and the second locking portion can be realized in a clamping manner, for example, the external thread is disposed on the outer wall of the first cover body 300, the internal thread is correspondingly disposed on the inner wall of the adsorption cavity 110, the first locking portion is conFIGured as a protrusion which protrudes toward the outer wall of the first cover body 300, a groove where the protrusion extends in is further correspondingly formed in the inner wall of the adsorption cavity 110, and when the first cover body 300 is screwed into the adsorption cavity 110 and the abutting surface 310 abuts against the filter part 200, the protrusion is clamped into the groove, thereby achieving locking between the first cover body 300 and the adsorption body 100; and when the first cover body 300 is screwed out of the adsorption cavity 110, the protrusion is separated from the groove.

As shown in FIG. 1 and FIG. 2, based on any one embodiment, the first cover body 300 is further provided with the blood nozzle 320 communicating with the adsorption cavity 110, and the abutting surface 310 is provided with the diversion channel 330 communicating with the blood nozzle 320. Thus, the liquid flows in the adsorption cavity 110 can be uniformly drained to the blood nozzle 320 via the diversion channel 330, thereby reducing the cavity volumes at two ends of the adsorption body 100 and distributing the liquid flows well. The liquid flows can flow through the adsorbent in the adsorption cavity 110 uniformly without dead angles.

The diversion channel 330 can be formed by either forming the division groove in the abutting surface 310 toward the abutting surface 310 or arranging a partition plate outside the abutting surface 310 on the abutting surface 310. In an embodiment, the abutting surface 310 is provided with at least two diversion parts 340 oppositely disposed at an interval, adjacent two diversion parts 340 are matched to form a runner, the runners communicate with each other to form a diversion channel 330, and the diversion part 340 abuts against the filter part 200 when the first cover body 300 is hermetically matched with the adsorption body 100. Thus, the runner is formed between at least two diversion parts 340 protruding out of the abutting surface 310, and the runners communicate with each other to drain the liquid flows uniformly to the blood nozzle 320. Meanwhile, the diversion part 340 is in contact with the filter part 200, thereby providing a supporting force to the filter part 200.

in an embodiment, the abutting surface 310 is provided with four annularly disposed diversion parts 340 at intervals, each diversion part 340 is disposed around the blood nozzle 320 and abuts against the filter part 200, the adjacent two diversion parts 340 are matched to form three runners, and each runner is provided with the notches 344, such that the three runners communicate one another to form the diversion channel 330 communicating with the blood nozzle 320. Thus, the three runners distribute the liquid flows uniformly and guide the flow liquids to the blood nozzle 320, and thereby, the liquid flows flow smoothly.

In an embodiment, the abutting surface 310 is provided with five annularly disposed diversion parts 340 at intervals, each diversion part 340 is disposed around the blood nozzle 320 and abuts against the filter part 200, the adjacent two diversion parts 340 are matched to form three runners, and each runner is provided with the notches 344, such that the four runners communicate one another to form the diversion channel 330 communicating with the blood nozzle 320. Thus, the four runners distribute the liquid flows uniformly and guide the flow liquids to the blood nozzle 320, and thereby, the liquid flows flow smoothly.

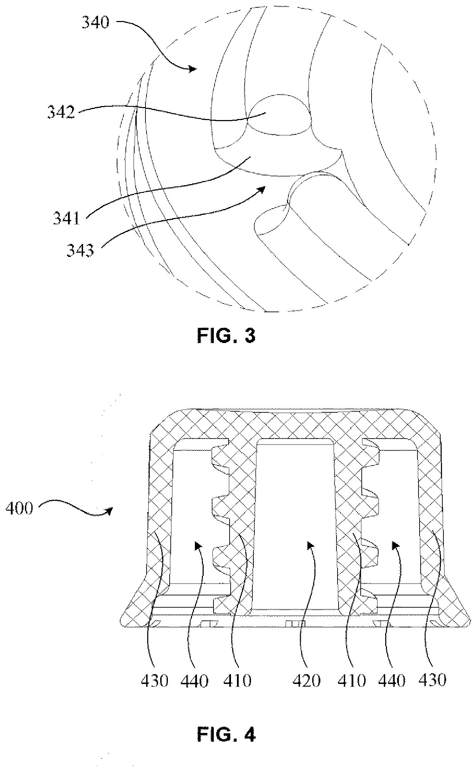

As shown in FIG. 2 and FIG. 3, the diversion part 340 can be a partition plate or a partition strip and it is only needed to guide flow of the liquid flows. In an embodiment, the diversion part 340 comprises the diversion seat 341 disposed on the abutting surface 310 and the diversion section 342 disposed on the diversion seat 341. Thus, the adjacent two diversion seats 341 and the adjacent two diversion sections 342 form runners, thereby draining the liquid flows. Further, a projection of the diversion section 342 on the abutting surface 310 falls into a projection of the diversion seat 341 on the abutting surface 310, i.e., a gap between the adjacent two diversion seats 341 is smaller than a gap between the adjacent two diversion sections 342, such that a flow rate of a part, close to diversion seat 341, of the liquid flow is higher than that of a part, close to the diversion section 342, of the liquid flow, such that the liquid flow can flush the bottom of the runner, thereby preventing the liquid flow from being blocked in the runner.

In an embodiment, the diversion part 340 is used for abutting against the filter part 200 and the diversion part 340 is in linear contact with the filter part 200. Thus, in order to decrease the contact area between the diversion part 340 and the filter part 200 and prevent the liquid flow from being left in a contact gap between the diversion part 340 and the filter part 200, such that the liquid flow can flow out or in smoothly. A contact part between the diversion part 340 and the filter part 200 is disposed in a curved surface or a cambered surface, for example, the diversion section 342 is disposed cylindrically, such that the diversion part 340 is in linear contact with the filter part 200.

As shown in FIG. 1 and FIG. 4, in addition, the packaging structure of the adsorbent further comprises the second cover body 400 which is hermetically matched with the first cover body 300 and seals the blood nozzle 320. Thus, the blood nozzle 320 is sealed by using the second cover body 400 and is prevented from being in contact with external air or foreign matters in a non-used state, thereby preventing the blood nozzle 320 from being polluted.

As shown in FIG. 2, further, the first cover body 300 is provided with the first mounting cavity 350, the bottom wall of the first mounting cavity 350 is provided with the blood nozzle 320 and the connectors 360 oppositely disposed on the outer wall of the blood nozzle at intervals, the connectors 360 are matched with the outer wall of the blood nozzle 320 to form the second mounting cavity 370 where the second cover body 400 is mounted. Thus, the second cover body 400 is mounted in the second mounting cavity 370, thereby isolating the blood nozzle 320 hermetically by the second cover body 400.

As shown in FIG. 2 and FIG. 4, in an embodiment, the connector 360 is disposed as a flange, the first thread is disposed on the outer wall, facing the second mounting cavity 370, of the flange, the second cover body 400 is disposed as a first sealing body 410 provided with the first sealing cavity 420, the outer wall of the first sealing body 410 is provided with a second thread in threaded fit with the first thread, and it is only needed to screw the first sealing body 410 into the second mounting cavity 370, such that the blood nozzle 320 extends into the first mounting cavity 420 to seal the blood nozzle 320.

As shown in FIG. 4, further, the first sealing body 410 is further circumferentially provided with the second sealing body 430, the second sealing body 430 is connected with the first sealing body 410 and the second sealing body and the first sealing body are disposed at an interval to form the second sealing cavity 440, and when the first sealing body 410 is screwed into the second mounting cavity 370, the flange also extends into the second sealing cavity 440, such that the risk that the blood nozzle 320 is in contact with outside can be further reduced. Further on, a seal ring can be further additionally disposed between the flange and the second sealing cavity 440 or a space between the flange and the second sealing cavity 440 can be further filled with a sealing medium, such that the blood nozzle 320 can be further prevented from being polluted.

As shown in FIG. 2, based on any one embodiment, the reinforcing rib 380 is further disposed on the inner wall of the first mounting cavity 350. Thus, the structural performance of the first cover body 300 can be improved and it is further convenient to screw the first cover body 300 into the adsorption cavity 110.

As shown in FIG. 1, based on any one embodiment, the packaging structure of the adsorbent further comprises the sealing part 500, the sealing part 50 is disposed in the adsorption cavity 110, and the sealing part 500, the first cover body 300 and the filter part 200 are matchable with each other to form a sealing structure for sealing the opening 111. Thus, the sealing structure is used to seal the adsorption cavity 110 tightly to prevent the adsorbent in the adsorption cavity 110 from being in contact with external air or foreign matters, thereby preventing the adsorbent from being polluted.

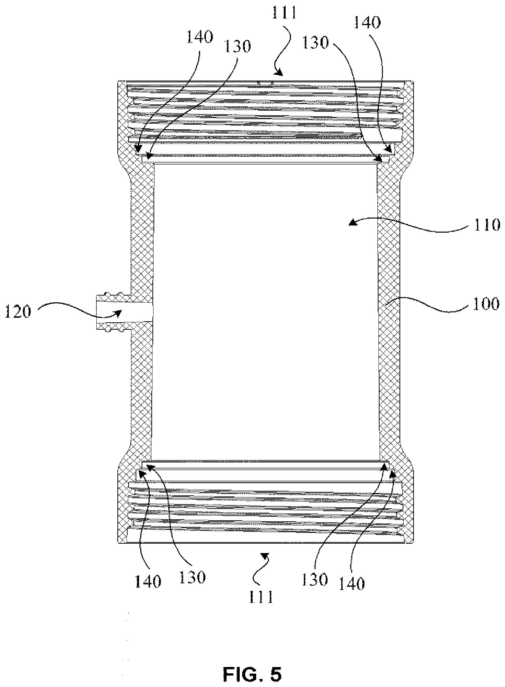

As shown in FIG. 1 and FIG. 5, in an embodiment, the inner wall of the adsorption cavity 110 is provided with the first step groove 130 and the second step groove 140 which are adjacent, the filter part 200 is disposed as a filter screen and is disposed in the net rack 210, the net rack 210 is disposed in the first step groove 130, the sealing part 500 is disposed as the seal ring, a part of the seal ring is disposed in the second step groove 140, the other part of the seal ring protrudes out of the second step groove 140 and abuts against the net rack 210, the edge of the abutting surface 310 of the first cover body 300 is provided with a sealing groove 390 matched with the seal ring, and after the first cover body 300 is screwed into the adsorption cavity 110, the bottom wall of the sealing groove 390 compresses the seal ring into the second step groove 140, such that the part, protruding out of the second step groove 140, of the seal ring is compressed to the net rack 210, and meanwhile, the part, close to the sealing groove 390, of the abutting surface 310 compresses the net rack 210 into the first step groove 130, the diversion part 340 on the abutting surface 310 is in contact with the filter screen, and the diversion part 340 further supports the filter screen. The seal ring can be made from a silica gel material with a good sealing property.

As shown in FIG. 1, in an embodiment, the disclosure further discloses a plasma adsorber, comprising any packaging structure of the adsorbent.

When the plasma adsorber of the embodiment is used, the adsorption cavity 110 is filled with the adsorbent which is packaged in the adsorption cavity 110 by using the filter part 200, and the filter part 200 and the adsorption body 100 are kept relatively fixed by hermetic fit of the first cover body 300 and the opening 111 of the adsorption body 100. When the liquid flow flows through, the filter part 200 can be kept stably, such that the adsorbent does not flow out along with flowing of the liquid flow reliably. Meanwhile, when the first cover body 300 is hermetically matched with the opening 111 of the adsorption body 100, the abutting surface 310 of the first cover body 300 is abutted against and attached to the filter part 200 or the filter part 200 is disposed on the abutting surface 310, such that the filter part 200 is disposed by being attached to the abutting surface 310, and then the first cover body 300 is hermetically matched with the opening 111 of the adsorption body 100. Therefore, even though the filter part 200 is subjected to the traction force along the direction of the liquid flow, the abutting surface 310 further can support the filter part 200, such that the traction force to the filter part 200 can be counteracted or alleviated, the filter part 200 can be prevented from being broken or falling off and the adsorbent is prevented from entering into the body of the patient, such that potential safety hazards are eliminated. Therefore, the plasma adsorber can complete the plasma adsorption process safely and reliably.

The packaging material of the adsorbent is made from a material meeting related requirements on medical apparatus and instruments, preferably, polypropylene or polycarbonate (PC).

The technical features of the above embodiments can be arbitrarily combined. To make the description concise, all possible combinations of the technical features in the above embodiments are not described. However, as long as there is no contradiction in the combination of these technical features, it should be considered as the scope of the description.

The above embodiments only express several embodiments of the disclosure, and the description is specific and detailed, but it can not be understood as a constraint on the scope of the disclosure. It should be pointed out that for ordinary skill in the art, on the premise of not departing from the concept of the disclosure, a number of deformations and improvements can be made, which belong to the protection scope of the disclosure. Therefore, the scope of protection of the disclosure shall be subject to the attached claims.

## Claims

1. A packaging structure of an adsorbent, **characterized by** comprising:
an adsorption body, the adsorption body comprising an adsorption cavity which is used for accommodating the adsorbent and comprises an opening;
a filter part; and
a first cover body, the first cover body comprising an abutting surface disposed toward the filter part and being hermetically matchable with the opening to abut the abutting surface with the filter part; or the filter part being disposed on the abutting surface, and the filter part being attached to the abutting surface.

2. The packaging structure of claim 1, **characterized in that** the first cover body is further provided with a blood nozzle communicating with the adsorption cavity, and the abutting surface is provided with a diversion channel communicating with the blood nozzle.

3. The packaging structure of claim 2, **characterized in that** the abutting surface is provided with at least two oppositely disposed diversion parts at intervals; adjacent two diversion parts are matched to form a runner, and runners communicate with each other to form the diversion channel; and the diversion part abuts against the filter part when the first cover body is hermetically matched with the adsorption body.

4. The packaging structure of claim 3, **characterized in that** the diversion part comprises a diversion seat disposed on the abutting surface and a diversion section disposed on the diversion seat.

5. The packaging structure of claim 2, **characterized in that** the diversion part is used for abutting against the filter part and the diversion part is in linear contact with the filter part.

6. The packaging structure of claim 2, further comprising a second cover body which is hermetically matched with the first cover body and seals the blood nozzle.

7. The packaging structure of claim 6, **characterized in that** the first cover body comprises a first mounting cavity, a bottom wall of the first mounting cavity is provided with the blood nozzle and connectors oppositely disposed on an outer wall of the blood nozzle at intervals, and the connectors are matched with the outer wall of the blood nozzle to form a second mounting cavity where the second cover body is mounted.

8. The packaging structure of claim 1, further comprising a sealing part, wherein the sealing part is disposed in the adsorption cavity, and the sealing part, the first cover body and the filter part are matchable with each other to form a sealing structure for sealing the opening.

9. The packaging structure of any one of claims 1-8, **characterized in that** an outer wall of the first cover body is provided with a first locking portion, and an inner wall of the adsorption cavity is provided with a second locking portion in locking fit with the first locking portion.

10. A plasma adsorber, **characterized by** comprising the packaging structure of the adsorbent of any one of claims 1-9.

## Patentansprüche

1. Verpackungsstruktur eines Adsorptionsmittels, **dadurch gekennzeichnet, dass** sie Folgendes umfasst:
einen Adsorptionskörper, wobei der Adsorptionskörper einen Adsorptionshohlraum umfasst, der verwendet wird, um das Adsorptionsmittel aufzunehmen und eine Öffnung umfasst,
einen Filterteil; und
einen ersten Abdeckkörper, wobei der erste Abdeckkörper eine Anlagefläche umfasst, die zu dem Filterteil angeordnet ist und hermetisch mit der Öffnung kombinierbar ist, um die Anlagefläche mit dem Filterteil in Anlage zu bringen; oder der Filterteil auf der Anlagefläche angeordnet ist und der Filterteil an der Anlagefläche angebracht ist.

2. Verpackungsstruktur nach Anspruch 1, **dadurch gekennzeichnet, dass** der erste Abdeckkörper ferner mit einer Blutdüse bereitgestellt ist, die mit dem Adsorptionshohlraum kommuniziert, und die Anlagefläche mit einem Umleitungskanal bereitgestellt ist, der mit der Blutdüse kommuniziert.

3. Verpackungsstruktur nach Anspruch 2, **dadurch gekennzeichnet, dass** die Anlagefläche mit zumindest zwei gegenüberliegend angeordneten Umleitungsteilen in Intervallen bereitgestellt ist, benachbarte zwei Umleitungsteile kombiniert sind, um einen Läufer zu bilden, und Läufer miteinander kommunizieren, um den Umleitungskanal zu bilden, und der Umleitungsteil an dem Filterteil anliegt, wenn der erste Abdeckkörper hermetisch mit dem Adsorptionskörper kombiniert ist.

4. Verpackungsstruktur nach Anspruch 3, **dadurch gekennzeichnet, dass** der Umleitungsteil einen Umleitungssitz, der auf der Anlagefläche angeordnet ist, und eine Umleitungssektion, der auf dem Umleitungssitz angeordnet ist, umfasst.

5. Verpackungsstruktur nach Anspruch 2, **dadurch gekennzeichnet, dass** der Umleitungsteil zum Anliegen an dem Filterteil verwendet wird und der Umleitungsteil in linearem Kontakt mit dem Filterteil ist.

6. Verpackungsstruktur nach Anspruch 2, ferner umfassend einen zweiten Abdeckkörper, der hermetisch mit dem ersten Abdeckkörper kombiniert ist und die Blutdüse abdichtet.

7. Verpackungsstruktur nach Anspruch 6, **dadurch gekennzeichnet, dass** der erste Abdeckkörper einen ersten Montagehohlraum umfasst, eine Bodenwand des ersten Montagehohlraums mit der Blutdüse und Anschlüssen, die gegenüberliegend an einer Außenwand der Blutdüse in Intervallen angeordnet sind, bereitgestellt ist, und die Anschlüsse mit der Außenwand der Blutdüse kombiniert sind, um einen zweiten Montagehohlraum zu bilden, in dem der zweite Abdeckkörper montiert ist.

8. Verpackungsstruktur nach Anspruch 1, ferner umfassend einen Dichtungsteil, wobei der Dichtungsteil in dem Adsorptionshohlraum angeordnet ist und der Dichtungsteil, der erste Abdeckkörper und der Filterteil miteinander kombinierbar sind, um eine Dichtungsstruktur zum Abdichten der Öffnung zu bilden.

9. Verpackungsstruktur nach einem der Ansprüche 1-8, **dadurch gekennzeichnet, dass** eine Außenwand des ersten Abdeckkörpers mit einem ersten Verriegelungsabschnitt bereitgestellt ist und eine Innenwand des Adsorptionshohlraums mit einem zweiten Verriegelungsabschnitt in Verriegelungspassung mit dem ersten Verriegelungsabschnitt bereitgestellt ist.

10. Plasmaadsorber, **dadurch gekennzeichnet, dass** er die Verpackungsstruktur des Adsorptionsmittels nach einem der Ansprüche 1-9 umfasst.

## Revendications

1. Structure de conditionnement d'un adsorbant, **caractérisée en ce qu'**elle comprend :
un corps d'adsorption, le corps d'adsorption comprenant une cavité d'adsorption qui est utilisée pour loger l'adsorbant et comprend une ouverture ;
une partie filtre ; et
un premier corps de couvercle, le premier corps de couvercle comprenant une surface de butée disposée vers la partie filtre et pouvant s'associer hermétiquement à l'ouverture pour mettre en butée la surface de butée avec la partie filtre ; ou la partie filtre étant disposée sur la surface de butée, et la partie filtre étant fixée à la surface de butée.

2. Structure de conditionnement selon la revendication 1, **caractérisée en ce que** le premier corps de couvercle est en outre pourvu d'une buse à sang communiquant avec la cavité d'adsorption, et la surface de butée est pourvue d'un canal de dérivation communiquant avec la buse à sang.

3. Structure de conditionnement selon la revendication 2, **caractérisée en ce que** la surface de butée est pourvue d'au moins deux parties de dérivation disposées de manière opposée à des intervalles ; deux parties de dérivation adjacentes sont associées pour former une goulotte, et les goulottes communiquent entre elles pour former le canal de dérivation ; et la partie de dérivation vient en butée contre la partie filtre lorsque le premier corps de couvercle est hermétiquement associé au corps d'adsorption.

4. Structure de conditionnement selon la revendication 3, **caractérisée en ce que** la partie de dérivation comprend un siège de dérivation disposé sur la surface de butée et une section de dérivation disposée sur le siège de dérivation.

5. Structure de conditionnement selon la revendication 2, **caractérisée en ce que** la partie de dérivation est utilisée pour venir en butée contre la partie filtre et la partie de dérivation est en contact linéaire avec la partie filtre.

6. Structure de conditionnement selon la revendication 2, comprenant en outre un second corps de couvercle qui est hermétiquement associé au premier corps de couvercle et scelle la buse à sang.

7. Structure de conditionnement selon la revendication 6, **caractérisée en ce que** le premier corps de couvercle comprend une première cavité de montage, une paroi inférieure de la première cavité de montage est pourvue de la buse à sang et de connecteurs disposés de manière opposée sur une paroi extérieure de la buse à sang à intervalles réguliers, et les connecteurs sont associés à la paroi extérieure de la buse à sang pour former une seconde cavité de montage où le second corps de couvercle est monté.

8. Structure de conditionnement selon la revendication 1, comprenant en outre une partie d'étanchéité, dans laquelle la partie d'étanchéité est disposée dans la cavité d'adsorption, et la partie d'étanchéité, le premier corps de couvercle et la partie filtre peuvent être associés les uns aux autres pour former une structure d'étanchéité destinée à sceller l'ouverture.

9. Structure de conditionnement selon l'une quelconque des revendications 1 à 8, **caractérisée en ce qu'**une paroi extérieure du premier corps de couvercle est pourvue d'une première portion de verrouillage, et une paroi intérieure de la cavité d'adsorption est pourvue d'une seconde portion de verrouillage en ajustement de verrouillage avec la première portion de verrouillage.

10. Adsorbeur à plasma, **caractérisé en ce qu'**il comprend la structure de conditionnement de l'adsorbant selon l'une quelconque des revendications 1 à 9.
